# EUROPEAN PATENT SPECIFICATION

(11) **EP 1 865 054 B1**
(45) Date of publication and mention of the grant of the patent: **06.10.2010**
(21) Application number: 07011018.4
(22) Date of filing: 05.06.2007
(51) Int. Cl.: C12N 9/08, C12N 9/96, C12N 9/99, C12Q 1/28, G01N 33/52

(54) **Inhibition of peroxidase enzymatic activity**
Hemmung der enzymatischen Peroxidaseaktivität
Inhibition de l'activité enzymatique d'une peroxidase

(30) Priority: 09.06.2006 EP 06011949
(43) Date of publication of application: 12.12.2007
(73) Proprietor: Roche Diagnostics GmbH, 68305 Mannheim (DE); F. Hoffmann-La Roche AG, 4070 Basel (CH)
(72) Inventor: Buchberger, Bernd, 82380 Peissenberg (DE); Kirr, Claudia, 82407 Wielenbach (DE); Watzele, Manfred, 82362 Weilheim (DE)

(56) References cited:
- US-A1- 4 448 882
- US-A1- 5 294 535
- US-A1- 2004 219 620

## Description

### Background of the Invention

A peroxidase is an enzyme, which may contain heme, that catalyzes a reaction of the form:

ROOR' + electron donor (2 e⁻) + 2H⁺ → ROH + R'OH

For many of these enzymes the optimal ROOR' (electron acceptor) substrate is hydrogen peroxide, but others are more active with organic hydroperoxides such as lipid peroxides.

The nature of the electron donor is very dependent on the structure of the enzyme. For example, horseradish peroxidase (HRP) which is isolated from horseradish (Armoracia rusticana) roots can use a variety of organic compounds as electron donors and acceptors.

Being a member of the ferroprotoporphyrin group of peroxidases, HRP does contain a heme group. Located at positions distal and proximal to the heme plane there are in addition two calcium binding sites. HRP C dominates quantitatively among the isoperoxidases of horseradish root. HRP C is a single chain polypeptide comprising 308 amino acid residues which form four internal disulfide bridges and 8 neutral carbohydrate side-chains. The molecular weight of the polypeptide chain is 33890 Daltons (Da), and the molecular weight of native horseradish peroxidase C is about 44 kDa (Welinder, K.G., Eur. J. Biochem. 96 (1979) 483-502). At least seven isozymes of HRP exist (Shannon, L.M., et al., J. Biol. Chem. 241 (1966) 2166-2172). The carbohydrate composition consists of galactose, arabinose, xylose, fucose, mannose, mannosamine, and galactosamine, depending upon the specific isozyme (Shannon, L.M., et al., J. Biol. Chem. 241 (1966) 2166-2172). The isoelectric point of the isozymes ranges from 3.0 to 9.0.

The pH optimum of HRP is in the range of pH 6.0 to pH 6.5; activity at pH 7.5 is 84% of the maximum. The enzyme is most stable in the range of pH 5.0 to pH 9.0 (Schomburg, D., et al., Enzyme Handbook 7 (1994) EC 1.11.1.7:1-6).

HRP combines with hydrogen peroxide (H₂O₂) and the resultant [HRP-H₂O₂] complex can oxidize a wide variety of chromogenic hydrogen donors. HRP has a broad and accessible active site and many chemically very different compounds can reach the site of the reaction. Known HRP substrates include TMB (3,3',5,5'-tetramethylbenzidine), ABTS (2,2'-azino-di-(3-ethylbenzthiazoline-6-sulphonic acid diammonium salt), luminol (5-amino-1,2,3,4-tetrahydrophthalazin-1,4-dion) and isoluminol (4-aminophthalhydrazide), as well as fluorogenic substrates such as tyramine (4-hydroxy-phenethylamine), homovanillic acid, and 4-hydroxyphenyl acetic acid. Further HRP substrates are known to the art. Due to its versatility, HRP is commercially used as a component of immunoassays, such as coupled enzyme assays, chemiluminescent assays and assay kits for clinical diagnostics including histochemistry kits.

In a typical example, an immunoassay based on a sandwich ELISA principle and using analyte-specific coating and capture antibodies includes HRP conjugated to the capture antibody. The peroxidase enzyme catalyzes the cleavage of a chromogenic substrate to yield a product which can be measured spectrophotometrically. The absorbance of the a colored or fluorescent product is directly correlated to the amount of analyte in the sample analyzed. In order to allow the comparison of simultaneous measurements including controls, the HRP enzymatic reaction needs to be terminated after a defined incubation period. To this end,a stop reagent is used. Stop reagents have to fulfil two major requirements: (1) to terminate the reaction by effectively inhibiting the enzymatic activity of HRP; (2) to stabilize the oxidized products of the chromogenic or fluorogenic substrate(s).

In Zollner, H., Handbook of Enzyme Inhibitors, 2nd Ed. (1989) Part A: 367-368) the following compounds have been described as inhibitors of HRP: sodium azide, cyanide, L-cystine, dichromate, ethylenethiourea, hydroxylamine, sulfide, vanadate, p-aminobenzoic acid, Cd⁺², Co⁺², Cu⁺², Fe⁺³, Mn⁺², Ni⁺², Pb⁺². Many stop reagents known to the art make use of these compounds. Another known reagent used to stop the HRP activity is oxalic acid.

When designing a stop reagent for reactions catalyzed by HRP, bleaching of the color brought about by the chromogenic substance is a frequent problem. The use of heavy metal salts in stop reagents has a number of disadvantages including toxicity. Also, certain salts of heavy metals are explosive as dry materials. Another known stop reagent for the HRP reaction is formaldehyde. However, the stabilizing effect of this compound is unsatisfactory. In addition, formaldehyde is toxic and has a troublesome smell.

US 4,234,680 teaches the use of an alkali metal bisulphite as stop reagent. However, this reducing agent has the potential of decolorizing the oxidized products formed from certain chromogenic substrates by the HRP catalyzed reaction. This applies especially to the oxidized products of ABTS and related salts.

In US 4,752,570 a process for the determination of peroxidase is described, wherein a chromogenic substrate reaction is stoped by adding catalase as stop reagent. This process, however, requires the use of an enzyme with limited stability and causes foaming due to the release of oxygen. Foaming may interfere with spectrophotometric readings.

Finally, surface-active agents, such as secondary alkyl sulphate or dodecyl hydrogen sulphate, have been suggested as stop agents. For example, SDS (sodium dodecyl sulphate) at a final concentration in solution of 0.5% [w/v] is suggested for stoping the color formation using HRP and ABTS, see pack insert of product #11684302 (catalogue of Roche Diagnostics GmbH, Mannheim, Germany). Similiarly, an SDS solution is supplied from KPL laboratories for the same purpose. However, these compounds are not able to fully suppress further color formation and/or lead to precipitation of the substrate. Results presented in Example 1 illustrates this fact.

Also strong (mineral) acids are used as stop reagents to inhibit peroxidase enzymatic activity. This is especially the case when 3,3'-5,5'-tetramethylbenzidine serves as a color substrate and the peroxidase enzyme is inhibited ba adjusting the pH to values of pH 2 or even lower. US 5,294,535 discloses the use of sulfuric acid for inhibiting peroxidase enzymatic activity in an aqueous solution comprising HRP enzyme, TMB as an electron acceptor substrate and urea peroxide as an electron donor substrate. US 4,448,882 teaches the use of of hydrochloric acid for the inhibition of peroxidase enzymatic activity. US 2004-219620 describes the use of an acidic stop reagent comprising a mineral acid such as hydrochloric, sulfuric and phosphoric acid.

Several reagents that are described above lead to precipitation of colored substrates like ABTS and cause erroneous readings, e.g. spectrophotometric readings of ELISA plates. Precipitation particularly occurs when using strong acids at pH values lower than 2. In addition, 1 M H₂SO₄ changes the color of the substrate 3,3'-5,5'-tetramethylbenzidine from blue to yellow. Other inhibitors (e.g. bisulphate, see above) may lead to bleaching and therefore are of limited use. Furthermore, certain stop reagents only after a certain lag phase achieve inhibition or just lead to an incomplete inhibition of the HRP-catalyzed reaction.

It is an object of the present invention to overcome the disadvantages of the stop reagents of the state of the art. It is a particular object of the present invention to provide an improved stop reagent for HRP-catalyzed reactions. Another object of the invention is to provide a process for the determination of peroxidase enzymatic activity in a sample.

### Summary of the Invention

A first embodiment of the invention is the use of (i) sodium dodecyl sulfate (SDS) at a concentration between 0.25% and 2.5% measured as weight by volume, and (ii) a pH value in the range of pH 2.5 to pH 4.2 for inhibiting peroxidase enzymatic activity in an aqueous solution comprising (a) a peroxidase enzyme, (b) an electron acceptor substrate, and (c) an electron donor substrate.. The invention further discloses a composition comprising (a) a peroxidase enzyme, (b) an electron acceptor substrate, (c) an electron donor substrate, and (d) a protein denaturing agent, characterized in that the composition is an acidic aqueous solution. Yet, a further embodiment of the invention is a method to inhibit peroxidase enzymatic activity in an aqueous solution comprising a peroxidase enzyme, an electron acceptor substrate, and an electron donor substrate, characterized in that the composition is mixed with an acidic aqueous solution of SDS, whereby in the resulting mixture the SDS concentration is between 0.25% and 2.5% measured as weight by volume, and the pH is adjusted to a final value of pH 2.5 to pH 4.2. The invention further discloses a kit comprising (a) a conjugate comprising horseradish peroxidase, (b) an acidic solution containing a protein denaturing agent. Yet, a further embodiment of the invention is a method to determine the amount of peroxidase enzymatic activity in a sample, comprising the steps of (a) adding to the sample an electron acceptor substrate and an electron donor substrate, whereby the electron donor substrate forms a dye or a pigment upon oxidation; (b) incubating the sample, whereby the peroxidase enzymatic activity catalyzes the oxidation of the electron donor substrate; (c) inhibiting the peroxidase enzymatic activity by way of mixing with the sample an acidic aqueous solution of SDS, whereby the SDS concentration in the mixture is between 0.25% and 2.5% measured as weight by volume, and the pH value of the mixture is adjusted at a pH in the range of pH 2.5 to pH 4.2; (d) determining the amount of dye or pigment formed; (e) correlating the amount of dye or pigment with the amount of peroxidase enzymatic activity in the sample.

### Detailed Description of the Invention

Certain terms are used with particular meaning, or are defined for the first time, in this description of the present invention. For the purposes of the present invention, the terms are defined by their art-accepted definitions, when such exist, except that when those definitions conflict or partially conflict with the definitions set forth below. In the event of a conflict in definition, the meaning of the terms are first defined by the definitions set forth below.

The term "comprising" is used in the description of the invention and in the claims to mean "including, but not necessarily limited to".

Horseradish peroxidase is also referred to as "HRP". Generally, the mixture of horseradish peroxidase isoenzymes isolated from horseradish roots is within the meaning of the term HRP. However, the term HRP also encompasses enriched, isolated, or recombinantly produced HRP C isoenzyme. Peroxidase (particularly HRP) activity can be assayed in a "reaction mixture" which comprises in an aqueous solution (a) a peroxidase enzyme, (b) an electron acceptor substrate, and (c) an electron donor substrate, whereby the salt content and the pH of the aqueous solution permit the peroxidase enzyme to catalyze the transfer of electrons from the electron donor substrate to the electron acceptor substrate. Regarding the electron donor substrate the invention generally contemplates compounds which upon oxidation form a dye or a pigment. Thus, the invention encompasses stop reagents for peroxidase-catalyzed reactions using (1) chromogenic, (2) fluorogenic, and (3) light emitting electron donor substrates. Such substrates are well known to the art.

As used herein, the term "stop reagent" refers to a reagent for terminating or inhibiting in a reaction mixture the chemical reaction catalyzed by HRP. In addition, the stop reagent stabilizes the chemical entities formed by the chemical reaction catalyzed by HRP, so that the amount of oxidized reaction product(s) of the reaction can be determined following the addition of the stop reagent to the reaction mixture. It is understood that the stop reagent according to the invention itself usually comprises a mixture of different compounds.

According to the present invention, a "protein denaturing agent" or "denaturing agent" is a chemical compound capable of denaturing proteins in aqueous solution. Denaturation is usually effected by changing the secondary or tertiary structure of the protein, particularly HRP. Thus, certain detergents are capable of changing the structure of HRP. The same result can be obtained by changing the interaction of the protein (HRP) with the solvent (water), e.g. by way of adding chaotropic substances. A high concentration of a chaotropic substance changes the bulk properties of water (Cacace, M.G., et al., Quarterly Review of Biophysics 30 (1997) 241-277) by weakening hydrophobic interactions, thereby causing proteins to denature. Whether effected by a detergent or a chaotropic substance, denaturation is not necessarily quantitative. Denaturation may be dependent, e.g. on the concentration of the protein denaturing agent.

The invention discloses the use of an acidic aqueous solution of a protein denaturing agent as a stop reagent to inhibit peroxidase enzymatic activity. A first embodiment of the invention is the use of (i) sodium dodecyl sulfate (SDS) at a concentration between 0.25% and 2.5% measured as weight by volume, and (ii) a pH value in the range of pH 2.5 to pH 4.2 for inhibiting peroxidase enzymatic activity in an aqueous solution comprising (a) a peroxidase enzyme, (b) an electron acceptor substrate, and (c) an electron donor substrate. As the skilled person appreciates, the stop reagent of the present invention is capable of stopping of the color formation in a peroxidase reaction mixture at a definite, predetermined point of time. The stop reagent of the invention is immediately effective and does not result in a disadvantageous change of the color present at the point of time of its addition to the reaction mixture.

The stop reagent of the invention comprises at least two essential components, wherein the first essential component shifts the pH of the reaction mixture to a value between 2.5 and 4.2, but without leading to the precipitation of the reaction product. That is to say, the reaction mixture is acidified mildly thereby avoiding precipitation of any member of the reaction mixture. According to the invention an aqueous solution comprising a protein denaturing agent is used to inhibit peroxidase enzymatic activity, whereby, the pH of the aqueous solution is preferably between pH 2.5 and pH 4.2. More preferred, the pH is between 2.7 and 3.8, even more preferred, between pH 3 and pH 3.5. Another preferred pH range of the aqueous solution is between pH 2.5 and pH 3. Yet, another preferred pH range of the aqueous solution is between pH 3 and pH 4.2.

The second essential component of the stop reagent is a protein denaturing agent which preferably is a detergent or a chaotropic substance. According to the invention, the protein denaturing agent is SDS. Further disclosed is a detergent selected from the group consisting of SDS (sodium dodecylsulfate), N-Laurylsarcosin, cetyltrimethylammonnium bromide (CTAB), and dodecyltrimethylamonnium bromide (DTAB) as well as mixtures thereof. Further disclosed is a chaotropic substance selected from the group consisting of urea or guanidinium hydrochloride, guanidinium isothioocyanate, and guanidinium thiocyanate, as well as mixtures thereof. Taken alone each of the said protein genaturing agents will not lead to a immediate and complete inhibition at the concentration applied. The surprising finding by the inventors was, however, that the combination of any one of the protein denaturants combined with an acidic pH leads to an exceptionally effective inhibition of peroxidase enzymatic activity.

The invention discloses a method to inhibit peroxidase enzymatic activity in an aqueous solution comprising a peroxidase enzyme, an electron acceptor substrate, and an electron donor substrate, characterized in that the composition is mixed with an acidic aqueous solution of a denaturing agent, whereby in the resulting mixture the pH is adjusted to a final value of pH 2.5 to pH 4.2. It is preferred that the final concentration of the denaturing agent is 0.25 to 10% weight by volume when the denaturing agent is a detergent. In case the denaturing agent is a chaotropic substance the preferred concentration is 5% to 70% weight by volume. According to the invention, in order to achieve very good HRP inactivation, peroxidase enzymatic activity in the reaction mixture is inhibited with SDS at a final concentration between 0.25% and 2.5% weight by volume. Thus, applying the method of the invention requires the skilled person to form a composition of the invention, the composition comprising (a) a peroxidase enzyme, (b) an electron acceptor substrate, (c) an electron donor substrate, and (d) a protein denaturing agent, characterized in that the composition is an acidic aqueous solution.

In a preferred embodiment, the electron donor substrate is a chromogenic, chemiluminescent or fluorogenic compound. The stop solution of the present invention can be used with the electron donor substrates known to the art for carrying out HRP determinations. Typical examples of frequently used electron donor substrates include 2,2'-azino-di-(3- ethylbenzthiazoline-6- sulphonic acid) diammonium salt (ABTS)®, 3,3'-5,5'-tetramethylbenzidine (TMB), 4-hydroxyphenethylamine (tyramine), 4-hydroxyphenyl acetic acid, homovanillic acid, o-phenylenediamine, p- phenylenediamine, m-aminosalicylic acid, dianisidine, p-aminobenzoic acid, aniline, 4-aminoantipyrine and the like. These chromogens are well known for HRP determinations and do not here require any further explanation.

Usually the electron acceptor substrate is hydrogen peroxide. However, other electron acceptor substrates are possible.

The present invention also discloses a process for the determination of peroxidase. An example therefor is an enzyme immunoassay in which an immobilized analyte is qualitatively or quantitatively detected by means of an antibody or antibody fragment conjugated to one or more peroxidase enzymes. In such a case the amount of bound peroxidase is proportional to the amount of analyte. Such an assay requires reacting the peroxidase with a peroxide and a substrate, and kinetic or end point measurement of the color resulting from the oxidation of the chromogen. In such assays color formation needs to be stoped after a definite period of time by the addition of a stop agent. Thus, the invention discloses a method to determine the amount of peroxidase enzymatic activity in a sample, comprising the steps of (a) adding to the sample an electron acceptor substrate and an electron donor substrate, whereby the electron donor substrate forms a dye or a pigment upon oxidation; (b) incubating the sample, whereby the peroxidase enzymatic activity catalyzes the oxidation of the electron donor substrate; (c) inhibiting the peroxidase enzymatic activity by way of mixing with the sample an acidic aqueous solution of SDS, whereby the SDS concentration in the mixture is between 0.25% and 2.5% measured as weight by volume, and the pH value of the mixture is adjusted at a pH in the range of pH 2.5 to pH 4.2; (d) determining the amount of dye or pigment formed; (e) correlating the amount of dye or pigment with the amount of peroxidase enzymatic activity in the sample.

The invention further discloses a kit comprising (a) a conjugate comprising horseradish peroxidase, (b) an acidic solution containing a protein denaturing agent. A preferred kit, according to the invention additionally comprises one or more microwell plates, a chromogenic, fluorogenic, or light emitting electron donor substrate, and an electron acceptor substrate. Also preferred, a kit contains peroxidase enzyme or an analyte binding agent (such as an antibody) conjugated to one or more peroxidase enzymes.

The following examples and figures are provided to aid the understanding of the present invention, the true scope of which is set forth in the appended claims. It is understood that modifications can be made in the procedures set forth without departing from the spirit of the invention.

### Description of the Figures

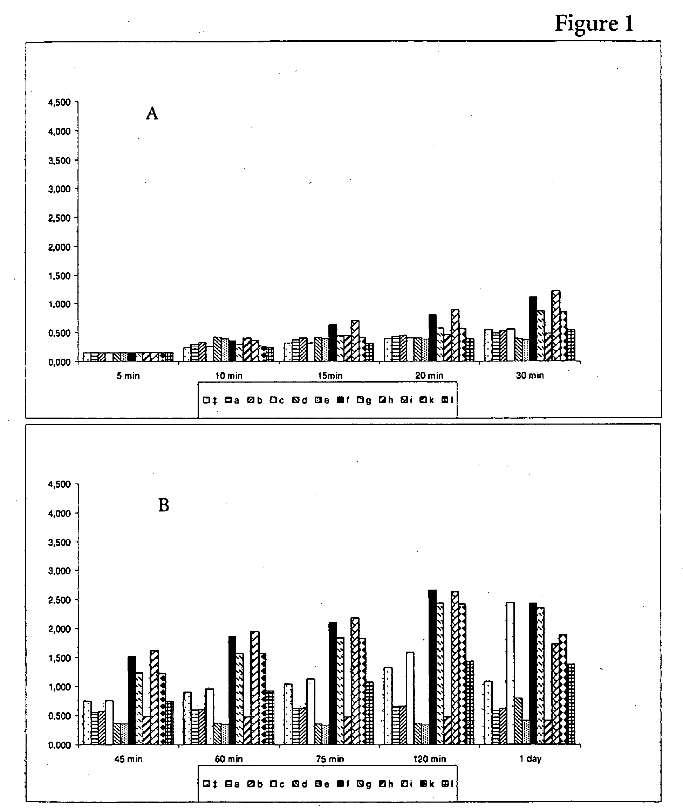

- **Figure 1**: **A and B:** inhibition of ABTS turnover catalyzed by a low amount of HRP (detection of a low amount of mono- and oligosomes in Jurkat cells; further details are given in Example 1). The ordinate indicates absorbance. Groups of bars represent comparative measurements at time points after the addition of stop reagents as indicated: (‡) - control, ABTS solution incubated without HRP, no stop reagent added; (a) to (k) - ABTS solution incubated with HRP and subsequently mixed with stop reagent as indicated: (a) 1% SDS, (b) 5% SDS, (c) distilled water, (d) 0.5 M H₂SO₄, (e) 0.25 M H₂SO₄, (f) 0.1 M H₂SO₄, (g) 0.05 M H₂SO₄, (h) 0.25 M oxalic acid, (i) 0.1 M oxalic acid, (k) 0.05 M oxalic acid, (1) distilled water.

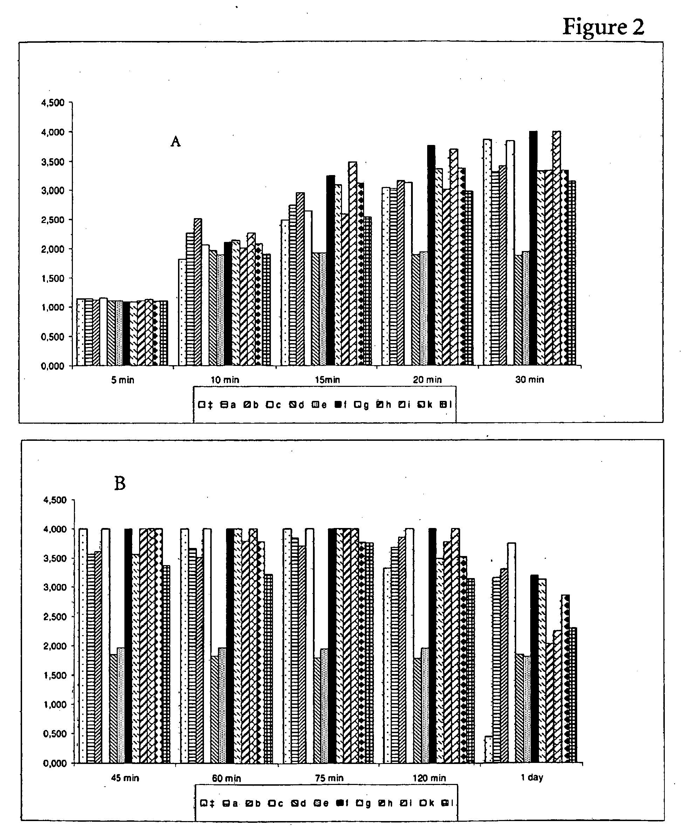

- **Figure 2**: **A and B**: inhibition of ABTS turnover catalyzed by a high amount of HRP detection of a high amount of mono- and oligosomes in Jurkat cells; further details are given in Example 1. All other designations are the same as in Figure 1.

### Example 1

### Inhibition of high and low amounts of HRP activity using SDS, sulfuric acid, and oxalic acid stop reagents

Jurkat cells were cultured and subsequentla divided into two culture dishes. In one dish the cells were treated with 1 µg/ml CAM (Camptothecin) to induce apoptosis, thereby leading to the formation of mono and oligonucleosomes, the target antigen. After an incubation period of 4 hours the cells of both dishes were harvested separately by centrifugation, lysed by the detergent contained in the Cell Death Detection ELISA Plus (Roche Diagnostics GmbH, Mannheim, Germany; Catalogue No. 1920685), and frozen. The mono and oligonucleosomes consisting of DNA and histones H2A, H2B, H3 and H4 were detected in thawed cell homogenate by way of a sandwich ELISA. Antigen was captured with a first monoclonal antibody against histone from the Cell Death Detection ELISA Plus kit. The antibody was coated onto the walls of a 96 well microplate. The detection antibody was a DNA-specific polyclonal antibody conjugated with horseradish peroxidase.

Non-induced and CAM-induced Jurkat cells yielded low and high amounts of target antigen, thereby leading to low and high absolute amounts of horseradish peroxidase enzymatic activity in the respective wells.

Into each well of the 96 well microplate an aliquot of 100 µl of ABTS® Solution (Roche Diagnostics GmbH, Mannheim, Germany; Roche Applied Science, product no. 11684302001) was added. After an incubation for 8 minutes at room temperature, 100 µl of either distilled water or a stop reagent as indicated in the legend to Figure 1 was added. Absorbance was measured at 405 nm at the time points indicated in Figure 1.

As can be seen in Figure 1, SDS and high concentrations of oxalic acid or sulfuric acid lead to a complete inhibition of the reaction. In Figure 2, however, it becomes clear that SDS and oxalic acid were not able to inhibit completely the high amount of HRP activity. In addition, H₂SO₄ and oxalic acid at high concentratios of 0.5 to 0.25M cause precipitation of the substrate.

### Comparative Example 2

### Inhibition of 1 mU of HRP activity using heavy metal salts and other compounds as stop reagents

Various described peroxidase inhibitor compounds as given in Table 1 were tested as stop reagents at the indicated concentrations with HRP in 100 µl of ABTS solution in a 96 well plate using a different experimental setting: HRP was added to the ABTS solution at an amount to yield an absolute peroxidase enzymatic activity of 1 mU per well. Stop reagent was added following an initial incubation of the ABTS / HRP mixture for 8 minutes at room temperature. For each stop reagent, several photometric readings were made after the time spans indicated in Table 2a and Table 2b. In these tables typical results of inhibition assays with the inhibitors of Table 1 are shown. When two different concentrations of an inhibitor were tested, only the result obtained with the higher concentration of the inhibitor was included in the tables. At the lower concentrations the inhibitors were not sufficiently effective when tested (data not shown).

**Table 1: Inhibitors of HRP tested**

| | Inhibitor: | 1st concentration | 2nd concentration |
|---|---|---|---|
| (i) | Manganese(II) carbonate | 0.25 mM | |
| (ii) | Lead(II) acetate | 0.5 M | |
| (iii) | Cobalt(II) acetate | 0.5 M | 0.05 M |
| (iv) | Cadmium(II) sulfate | 0.5 M | 0.05 M |
| (v) | Iron(III) nitrate | 0.5 M | |
| (vi) | Copper(II) sulfate | 0.5 M | 0.05 M |
| (vii) | Sodium orthovanadate | 0.5 M | 0.05 M |
| (viii) | NaF | 0.4 M | |
| (ix) | Nickel (II) chloride | 0.1 M | |
| (x) | Sodium azide | 0.25 M | 0.025 M |
| (xi) | Sodium Oxamate | 0.1 M | |
| (xii) | KSCN | 0.1 M | 0.05 M |
| (xiii) | NaOH | 0.5 M | 0.1 M |

It was also noted that vanadate, sodium hydroxide, potassium thiocyanate and sodium azide resulted in a decoloration of the dye, whereas only NaF and the commercially available stop reagent (designated "comm.." in Table 2b) effected inhibition, however only partially.

**Table 2a: Absorbance at 405 nm after time intervals as indicated**

| Time | ‡ | (i) | (ii) | (iii) | (iv) | (v) | (xiii) | (vi) |
|---|---|---|---|---|---|---|---|---|
| 6 min | 1,090 | 1,066 | 1,065 | 1,062 | 1,059 | 1,054 | 1,036 | 1,077 |
| 9 min | 1,413 | 1,560 | 1,864 | 1,471 | 1,759 | 3,999 | 0,878 | 1,646 |
| 15 min | 2,183 | 2,173 | 0,761 | 2,666 | 2,907 | 3,999 | 0,027 | 1,669 |
| 30 min | 3,255 | 3,260 | 1,900 | 2,666 | 3,999 | 3,999 | 0,027 | 1,767 |
| 45 min | 3,565 | 3,767 | 2,998 | 2,666 | 3,999 | 3,999 | 0,027 | 1,831 |
| 60 min | 3,763 | 3,999 | 3,999 | 2,666 | 3,999 | 3,999 | 0,027 | 1,765 |
| 180 min | 3,999 | 3,999 | 3,999 | n.d. [*] | 2,379 | 3,999 | 0,026 | 1,842 |

| | | | | | | | | |
|---|---|---|---|---|---|---|---|---|
| (‡) indicates the control: ABTS solution incubated without HRP, no stop reagent added. [*] negative value deleted. | | | | | | | | |

**Table 2b: Absorbance at 405 nm after time intervals as indicated**

| Time | ‡ | (vii) | (viii) | (ix) | (x) | (xi) | (xii) | comm. |
|---|---|---|---|---|---|---|---|---|
| 6 min | 1,189 | 1,173 | 1,176 | 1,175 | 1,172 | 1,182 | 1,179 | 1,159 |
| 9 min | 1,531 | 0,115 | 1,343 | 2,241 | 1,395 | 1,776 | 1,207 | 1,948 |
| 15 min | 2,459 | 0,096 | 1,424 | 2,999 | 0,111 | 2,525 | 1,290 | 2,453 |
| 30 min | 3,999 | 0,089 | 1,572 | 3,719 | 0,028 | 3,091 | 1,201 | 2,699 |
| 45 min | 3,999 | 0,088 | 1,721 | 3,999 | 0,026 | 3,097 | 1,086 | 2,704 |
| 60 min | 3,999 | 0,092 | 1,818 | 3,715 | 0,024 | 3,095 | 0,989 | 2,693 |
| 180 min | 3,999 | 0,100 | 2,107 | 3,999 | 0,024 | 2,865 | 0,849 | 2,634 |

| | | | | | | | | |
|---|---|---|---|---|---|---|---|---|
| (‡) indicates the control: ABTS solution incubated without HRP, no stop reagent added. "comm." indicates a commercially available stop reagent from KPL, Inc., Gaithersburg, MD, USA; catalogue no. 50-85-01 | | | | | | | | |

### Example 3

### Inhibition of 1 mU of HRP activity using acidified SDS solution as stop reagent

A solution of 5% [w/v] sodium dodecyl sulfate acidified with 0.25 M hydrochloric acid was used as a stop reagent to inhibit 1 mU peroxidase enzymatic activity of HRP. The experimental setting was similar to that in Example 2, using an amount of 1 mU of HRP activity in 100 µl of ABTS solution. 100 µl of stop reagent were added.

**Table 3: Absorbance at 405 nm after time intervals as indicated**

| | ‡ (control) | 5% SDS / 25mM HCl |
|---|---|---|
| 5 min | 1,134 | 1,157 |
| 7 min | 1,378 | 1,866 |
| 10 min | 1,824 | 1,853 |
| 15 min | 2,902 | 1,841 |
| 30 min | 3,999 | 1,833 |
| 45 min | 3,999 | 1,833 |
| 60 min | 3,999 | 1,837 |
| 120 min | 3,999 | 1,845 |

| | | |
|---|---|---|
| (‡) indicates the control: ABTS solution incubated without HRP, no stop reagent added. | | |

Another experiment was performed in which two different SDS concentrations, 5% and 0.5% [w/v] in an acidified solution were compared.

**Table 4: Absorbance at 405 nm after time intervals as indicated**

| | ‡ (control) | 5% SDS / 25mM HCl | 0.5% SDS / 25mM HCl |
|---|---|---|---|
| 5 min | 0,848 | 0,850 | 0,846 |
| 7 min | 1,122 | 1,288 | 1,178 |
| 10 min | 1,703 | 1,283 | 1,219 |
| 15 min | 2,507 | 1,284 | 1,246 |
| 30 min | 3,999 | 1,281 | 1,259 |
| 45 min | 3,745 | 1,273 | 1,250 |
| 60 min | 3,759 | 1,272 | 1,246 |

It was found that acidification of the reaction mixture in the well to a pH value of about 2.5 to 4.2 in combination with SDS at a final concentration of between 0.25% and 2.5% weight by volume is very effective in inhibiting peroxidase enzymatic activity. At the same time, the concentration of the acidic component does not cause precipitation of the substrate.

## Claims

1. Use of (i) sodium dodecyl sulfate (SDS) at a concentration between 0.25% and 2.5% measured as weight by volume, and (ii) a pH value in the range of pH 2.5 to pH 4.2 for inhibiting peroxidase enzymatic activity in an aqueous solution comprising
(a) a peroxidase enzyme,
(b) an electron acceptor substrate, and
(c) an electron donor substrate.

2. A method to inhibit peroxidase enzymatic activity in an aqueous solution comprising a peroxidase enzyme, an electron acceptor substrate, and an electron donor substrate, **characterized in that** the composition is mixed with an acidic aqueous solution of SDS, whereby in the resulting mixture the SDS concentration is between 0.25% and 2.5% measured as weight by volume, and the pH is adjusted to a final value of pH 2.5 to pH 4.2.

3. The method according to claim 2, **characterized in that** the concentration of SDS is 2.5%, measured as weight by volume.

4. The method according to claim 2, **characterized in that** the concentration of SDS is 0.25%, measured as weight by volume.

5. The method according to any of the claims 2 to 4, **characterized in that** the peroxidase is horseradish peroxidase (HRP).

6. The method according to claim 5, **characterized in that** 1 mU of HRP is inhibited.

7. The method according to any of the claims 5 and 6, **characterized in that** the aqueous solution contains 2,2'-azino-di-(3-ethylbenzthiazoline-6-sulphonic acid diammonium salt (ABTS).

8. A method to determine the amount of peroxidase enzymatic activity in a sample, comprising the steps of
(a) adding to the sample an electron acceptor substrate and an electron donor substrate, whereby the electron donor substrate forms a dye or a pigment upon oxidation;
(b) incubating the sample, whereby the peroxidase enzymatic activity catalyzes the oxidation of the electron donor substrate;
(c) inhibiting the peroxidase enzymatic activity by way of mixing with the sample an acidic aqueous solution of SDS, whereby the SDS concentration in the mixture is between 0.25% and 2.5% measured as weight by volume, and the pH value of the mixture is adjusted at a pH in the range of pH 2.5 to pH 4.2;
(d) determining the amount of dye or pigment formed;
(e) correlating the amount of dye or pigment with the amount of peroxidase enzymatic activity in the sample.

## Patentansprüche

1. Verwendung von (i) Natriumdodecylsulfat (SDS) mit einer Konzentration zwischen 0,25 % und 2,5 %, gemessen als Gewicht pro Volumen, und (ii) einem pH-Wert im Bereich von pH = 2,5 bis pH = 4,2 zum Hemmen von Peroxidase-Enzymaktivität in einer wässrigen Lösung, umfassend
(a) ein Peroxidaseenzym,
(b) ein Elektronenakzeptor-Substrat, und
(c) ein Elektronendonator-Substrat.

2. Verfahren zum Hemmen von Peroxidase-Enzymaktivität in einer wässrigen Lösung, umfassend ein Peroxidaseenzym, ein Elektronenakzeptor-Substrat und ein Elektronendonator-Substrat, **dadurch gekennzeichnet, dass** die Zusammensetzung mit einer sauren, wässrigen Lösung von SDS gemischt wird, wodurch die SDS-Konzentration in dem erhaltenen Gemisch zwischen 0,25 % und 2,5 %, gemessen als Gewicht pro Volumen, beträgt, und der pH-Wert auf einen Endwert von pH = 2,5 bis pH = 4,2 eingestellt wird.

3. Verfahren gemäß Anspruch 2, **dadurch gekennzeichnet, dass** die Konzentration von SDS 2,5 %, gemessen als Gewicht pro Volumen, beträgt.

4. Verfahren gemäß Anspruch 2, **dadurch gekennzeichnet, dass** die Konzentration von SDS 0,25 %, gemessen als Gewicht pro Volumen, beträgt.

5. Verfahren gemäß einem der Ansprüche 2 bis 4, **dadurch gekennzeichnet, dass** die Peroxidase Meerrettich-Peroxidase (HRP) ist.

6. Verfahren gemäß Anspruch 5, **dadurch gekennzeichnet, dass** 1 mU HRP gehemmt wird.

7. Verfahren gemäß einem der Ansprüche 5 und 6, **dadurch gekennzeichnet, dass** die wässrige Lösung 2,2'-Azinodi(3-ethylbenzthiazolin-6-sulfonsäure)diammoniumsalz (ABTS) enthält.

8. Verfahren zum Bestimmen der Menge an Peroxidase-Enzymaktivität in einer Probe, umfassend die Schritte
(a) Zugeben eines Elektronenakzeptor-Substrats und eines Elektronendonator-Substrats zu der Probe, wodurch das Elektronendonator-Substrat durch Oxidation einen Farbstoff oder ein Pigment bildet;
(b) Inkubieren der Probe, wodurch die Peroxidase-Enzymaktivität die Oxidation des Elektronendonator-Substrats katalysiert;
(c) Hemmen der Peroxidase-Enzymaktivität durch Mischen einer sauren, wässrigen Lösung von SDS mit der Probe, wodurch die SDS-Konzentration in dem Gemisch zwischen 0,25 % und 2,5 %, gemessen als Gewicht pro Volumen, beträgt und der pH-Wert des Gemischs auf einen pH-Wert im Bereich von pH = 2,5 bis pH = 4,2 eingestellt wird;
(d) Bestimmen der Menge an gebildetem Farbstoff oder Pigment;
(e) Korrelieren der Menge an Farbstoff oder Pigment mit der Menge an Peroxidase-Enzymaktivität in der Probe.

## Revendications

1. Utilisation de (i) dodécylsulfate de sodium (SDS) à une concentration comprise dans la plage allant de 0,25 % à 2,5 % mesurée en poids par volume, et (ii) une valeur de pH comprise dans la plage allant de pH 2,5 à pH 4,2 pour l'inhibition de l'activité enzymatique d'une peroxydase dans une solution aqueuse comprenant
(a) une enzyme peroxydase,
(b) un substrat accepteur d'électrons, et
(c) un substrat donneur d'électrons.

2. Procédé d'inhibition de l'activité enzymatique d'une peroxydase dans une solution aqueuse comprenant une enzyme peroxydase, un substrat accepteur d'électrons et un substrat donneur d'électrons, **caractérisé en ce que** la composition est mélangée avec une solution aqueuse acide de SDS, où dans le mélange résultant, la concentration en SDS est comprise dans la plage allant de 0,25 % à 2,5 % mesurée en poids par volume, et le pH est ajusté à une valeur finale comprise dans la plage allant de pH 2,5 à pH 4,2.

3. Procédé selon la revendication 2, **caractérisé en ce que** la concentration en SDS est de 2,5 %, mesurée en poids par volume.

4. Procédé selon la revendication 2, **caractérisé en ce que** la concentration en SDS est de 0,25 %, mesurée en poids par volume.

5. Procédé selon l'une quelconque des revendications 2 à 4, **caractérisé en ce que** la peroxydase est la peroxydase de raifort (HRP).

6. Procédé selon la revendication 5, **caractérisé en ce que** 1 mU de HRP est inhibée.

7. Procédé selon l'une quelconque des revendications 5 et 6, **caractérisé en ce que** la solution aqueuse comprend un sel de diammonium d'acide 2,2'-azino-di-3-éthylbenzthiazoline-6-sulfonique (ABTS).

8. Procédé de détermination de la quantité d'activité enzymatique d'une peroxydase dans un échantillon, comprenant les étapes consistant à
(a) ajouter à l'échantillon un substrat accepteur d'électrons et un substrat donneur d'électrons, le substrat donneur d'électrons formant un colorant ou un pigment lors de l'oxydation ;
(b) incuber l'échantillon, l'activité enzymatique de la peroxydase catalysant l'oxydation du substrat donneur d'électrons ;
(c) inhiber l'activité enzymatique de la peroxydase en mélangeant avec l'échantillon une solution aqueuse acide de SDS, la concentration en SDS dans le mélange étant comprise dans la plage allant de 0,25 % à 2,5 % mesurée en poids par volume, et le pH étant ajusté à une valeur finale comprise dans la plage allant de pH 2,5 à pH 4,2 ;
(d) déterminer la quantité de colorant ou de pigment formée ;
(e) établir une corrélation entre la quantité de colorant ou de pigment et la quantité d'activité enzymatique de la peroxydase dans l'échantillon.
